# EUROPEAN PATENT APPLICATION

(11) **EP 3 111 958 A1**
(43) Date of publication of application: **04.01.2017**
(21) Application number: 15461544.7
(22) Date of filing: 30.06.2015
(51) Int. Cl.: A61K 47/24, A61K 9/127, A61K 31/00

(54) **SUPRAMOLECULAR AGGREGATE COMPOSED OF PHOSPHATIDYLOCHOLINEA AND OCTENIDINE, COMPOSITIONS COMPRISING (CONTAINING) IT AND METOD FOR OBTAINING THEREOF**

(71) Applicant: Pharmbridge Sp. z o.o., 91-485 Lodz (PL)
(72) Inventor: Langner, Marek, 51-314 Wroc aw (PL); Przybylo, Magdalena, 51-314 Wroc aw (PL)
(74) Representative: Wroblewski, Michal

(57) **Abstract**

The present invention describes a supramolecular aggregate as a complex of octenidine and phosphatidylcholine, its composition and method of preparation. Phosphatidylcholine and octenidine forming supramolecular aggregates should be in a molecular ratio of from 2:1 to 1:2. Supramolecular aggregates in the form of octenidine and phosphatidylcholine complex may be included in suspensions and gels. Aggregates are produced by extruding the hydrated mixture of phosphatidylcholine and octenidine premixed in a solvent from the group of polyhydric alcohols with a low molecular weight.

## Description

The present invention is a supramolecular aggregate in the form of a phosphatidylcholine-octenidine complex with antiseptic qualities a supramolecular aggregate and the method of preparation thereof.

Antiseptics based on bipyridine alkanes and, in particular octenidine dihydrochloride (hereinafter referred to as octenidine) are highly effective in the treatment of skin infections, mucous membranes, and as an antiseptic agent.

For example the patent EP0411315A1 discloses an aqueous disinfectant solution comprising octenidine, phenoxypropanol, and/or phenoxyethanol. Patent DE19647692A1 describes a disinfectant solution containing octenidine, C₁-C₈ alkyl alcohol, nonionic or cationic surfactant and α-hydroxycarboxylic acid.

The product named Octenisept^{™} which has for years been sold by Schülke& MayrGmbH, Norderstedt, as an antiseptic for wounds and mucous membranes, comprises octenidine, phenoxyethanol, and cocamidopropylbetaine as amphoteric surfactant. The patent document EP1982696B1 refers to antiseptic gel based on octenidine and/or polyhexanide characterized by high viscosity at the temperature of 20 °C to 30°C.

Isotonic aqueous solution of antiseptic disclosed in the patent application DE10205883A1 comprises octenidine, nonionic surfactant and excipients. A suitable osmotic pressure is obtained by isotonic concentration of inorganic salts. This composition is useful for the preparation used for disinfection of skin, mucous membranes, wounds or internal organs. However, patent application DE102005058978A1 discloses aqueous solutions for disinfecting wounds and mucous membranes which comprise octenidine and one or more active ingredients selected from the group consisting of ethanol, 1-propanol, 2-propanol, methylammonium sulfate, N, N, N-trimethyl-3-((1-oxo-10-undecenylamino) propylamine, 3-(4-chlorophenoxy) -1,2-propanediol, sodium hydroxymethylglycinate, glycerin, 1,2-diols (C3-C10), and optionally, surfactants, emulsifiers, solubility promoters, pH adjusters, dyes, fragrances and/or thickening agents. Patent DE 60320087T2 describes an alcohol and aryloxy alcohol free antiseptic containing from 0.01% to 10% by weight of bispyridinium alkanes, preferably octenidine hydrochloride and 0.01% to 20 % by weight of nonionic surfactants, for use as a disinfectant of skin, mucous membranes, wounds and internal organs.

All disinfectants which are currently in use, including the ones disclosed in the above patents are not optimal for the following reasons:
- the alcohols used (aliphatic alcohols such as ethanol, propanol, butanol, or aromatic alcohols such as benzyl alcohol, phenoxyethanol, phenoxypropanol) can be irritant and may cause allergic reactions;
- the surfactants (cationic, anionic, nonionic, or amphoteric) used as auxiliary solubilizers for octenidine, may cause an allergic reaction and produce undesirable foam, especially when ultrasounds are used for wound healing.
- the bispyridinium alkanes used, such as octenidine are often unstable in the presence of nonionic surfactants, which requires the use of antioxidants that are undesirable in disinfectants, especially when applied to irritated or sensitive areas.
- the disinfectants used to treat wounds and mucous membranes hould be isotonic, as described in the patent DE60320087T2, which usually is accomplished by adding inorganic salts, which in turn can cause the precipitation of octenidine.

Patent EP1926473B1 discloses a liposomal form of octenidine produced by homogenization, which is devoid of most of the above-mentioned disadvantages. The protected lipid concentration is from 1 to 10%. However, the disclosed liposomal form of octenidine does have two shortcomings. The use of high energy homogenization may cause the liposome suspension to be unstable. Another disadvantage of the liposomal formulation of octenidine is high affinity of octenidine to the lipid fraction of liposomes, which means that the bactericidal effect will be reduced or even eliminated. This fact is implemented in neutralizers defined in the Polish norm PN-EN 123 727+A1 entitled: "Chemical disinfectants and antiseptics - Quantitative suspension test for the evaluation of bactericidal activity in the medical area - Test method and requirements" which eliminate the bactericidal effect of octenidine.

The first aspect of the invention is a supramolecular aggregate in the form of phosphatidylcholine-octenidine complex, wherein the molar ratio of octenidine to phosphatidylcholine ranges from 1:2 to 2:1, and the concentration of octenidine and phosphatidylcholine equals respectively from 0.05 to 2% by weight, and from 0.05 to 5% by weight. For phosphatidylcholine, this aggregate comprises phosphatidylcholine selected from the group consisting of soy phosphatidylcholine, rapeseed phosphatidylcholine, hen's eggs phosphatidylcholine.

The second aspect of the invention is a pharmaceutical composition in the form of a suspension comprising supramolecular aggregate of phosphatidylcholine and octenidine dissolved in the polyhydric alcohol of low molecular weight, wherein the molar ratio of octenidine to phosphatidylcholine ranges from 1: 2 to 2: 1, the concentration of octenidine equals from 0.05 to 2% by weight and the concentration of phosphatidylcholine equals from 0.05 to 5% by weight, wherein the concentration of propane-1,2-diol equals from 1 to 20% by weight.

The third aspect of the invention is a pharmaceutical composition in the form of a gel containing supramolecular aggregate of phosphatidylcholine and octenidine dissolved in the polyhydric alcohol of low molecular weight, wherein the molar ratio of octenidine to phosphatidylcholine ranges from 1: 2 to 2: 1, the concentration of octenidine equals from 0.05 to 2% by weight and the concentration of phosphatidylcholine equals from 0.05 to 5% by weight. As a gelling agent, this composition comprises an acrylic acid polymer, and as a preservative comprises preferably methyl parahydroxybenzoate.

Another aspect of the invention is the process for preparation of a supramolecular aggregate comprising phosphatidylcholine and octenidine, wherein the molar ratio of octenidine to phosphatidylcholine ranges from 1: 2 to 2: 1, the concentration of octenidine equals from 0.05 to 2% by weight and the concentration of phosphatidylcholine equals from 0.05 to 5% by weight. According to the present invention, octenidine is mixed with phosphatidylcholine in the presence of propane-1,2-diol, where upon the mixture is homogenized with water and/or water with a buffer, and then extruded through a polycarbonate filter pores with a diameter of 50 to 400 nm at the temperature of 20° do 40° C.

The buffer is selected from the group comprising triethanolamine and biological buffers. The biological buffer used is selected from the group consisting of 2- [4- (2-hydroxyethyl) -1-piperazinyl] ethanesulfonic acid (HEPES), 2-morpholineoethanesulfonic acid (MES), 3-[N-morpholino]propanesulfonic acid (MOPS), piperazine-1,4-bis[2-ethanesulfonic] acid (PIPES), N-[Tris(hydroxymethyl)methyl]-2-aminoethanesulfonic acid (TES), 1-hydroxy-4-sulfobenzoic acid (SSA). The buffer concentration does not exceed 5% by weight.

According to the invention, the supramolecular aggregate is devoid of all the disadvantages of formulations disclosed in the priorart. Phosphatidylcholine protects octenidine against oxidation and prevents its precipitation, resulting in a stable suspension in isotonic conditions. The netpositive charge of the supramolecular aggregate results in high affinity to the negatively charged bacterial membranes, which greatly increases its effectiveness in comparison to other formulations. In addition, the aggregate form of octenidine causes the formulation to work more efficiently than the true solution because the association of the supramolecular aggregate with the bacterial membrane or a virus introduces a large number of octenidine molecules at one time, resulting in destruction of the bacterial membrane or the viral envelope. The lack of membrane structures (liposomes) in the formulation does not restrict the functional availability of octenidine.

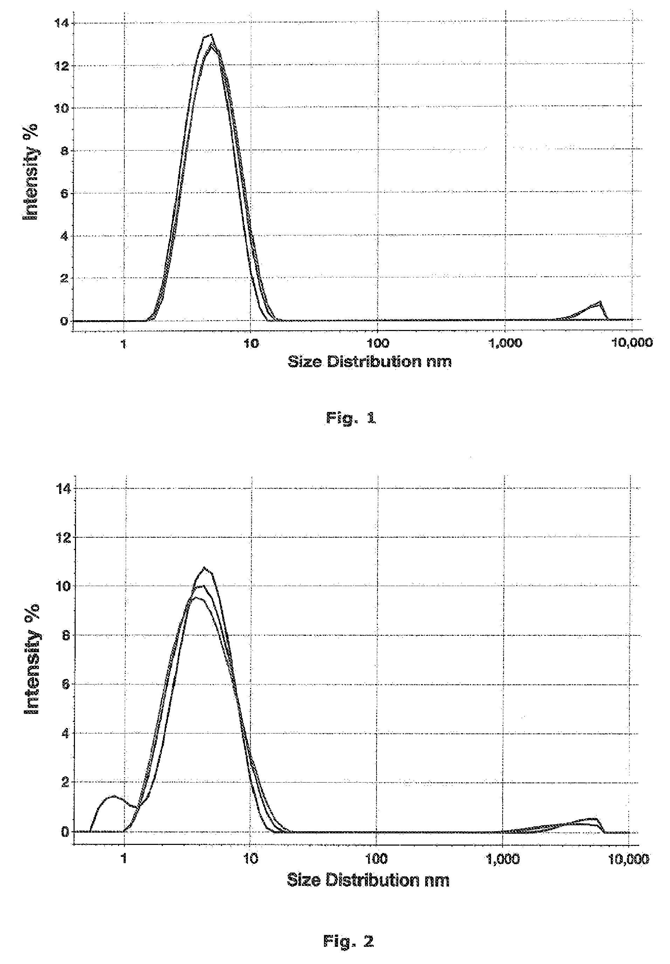

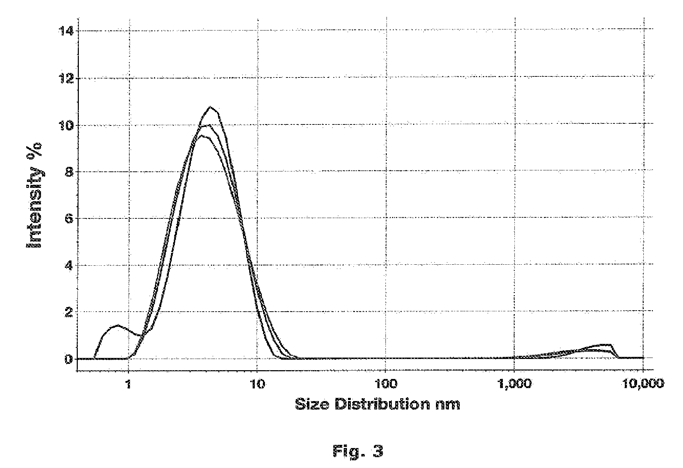

The invention is disclosed in embodiments and drawings without limiting the protection scope, wherein fig. 1 shows the size distribution of the supramolecular aggregates of phosphatidylcholine and octenidine according to Example 1, fig. 2 - shows supramolecular aggregates size distribution of phosphatidylcholine and octenidine according to Example 4, fig. 3 -shows supramolecular aggregates size distribution of phosphatidylcholine and octenidine according to example 6.

### Example 1.

14.4 g of soybean phosphatidylcholine 90G was premixed with 12.1 g of octenidine in the presence of 12.1 g of propane-1,2-diol (octenidine concentration of approx. 1 %).The mixture was then homogenized with 1000 ml of water and then extruded through polycarbonate filter pores with the diameter of 100 nm at the temperature of 20° C.

The particle size distribution of the supramolecular aggregate in the obtained suspension was estimated based on the dynamic light scattering technique. The measurements were performed with a laser beam having a wavelength λ=633 nm with a power of 4 mW at the angle of 173 degrees. Samples were analyzed immediately after preparation without dilution. The obtained supramolecular aggregates of phosphatidylcholine and octenidine had an average size of 3.94±0.04 nm with the PDI polydispersity factor of 0.208±0.06.

### Example 2.

28.8 g of soybean phosphatidylcholine 90G was premixed with 12.1 g of octenidine in the presence of 12.1 g of glycerin (octenidine concentration of approx. 1 %). The mixture was then homogenized with 1000 ml of water and then extruded through polycarbonate filter pores with a diameter of 400 nm at the temperature of 40° C. The ratio of octenidine to phosphatidylcholine is approximately 1: 2.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. Samples were analyzed immediately after preparation without dilution. The obtained supramolecular aggregates of phosphatidylcholine and octenidine had an average size of 6.63±0.21 nm with the PDI polydispersity factor of 0.213±0.011.

### Example 3.

7.2 g of rapeseed phosphatidylcholine was premixed with 12.06 g of octenidine in the presence of 12.1 g of propane-1,2-diol. The mixture was then homogenized with 1000 ml of water and then extruded through polycarbonate filter pores with the diameter of 50 nm at the temperature of 20° C. The ratio of octenidine to phosphatidylcholine is 2: 1.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. Samples were analyzed immediately after preparation without dilution. The obtained supramolecular aggregates of phosphatidylcholine and octenidine had an average size of 3.22±0.009 nm with the PDI polydispersity factor of 0.092±0.003. Example 4.

14.4 g of rapeseed phosphatidylcholine was premixed with 6.0 g of octenidine in the presence of 12.1 g of propane-1,2-diol (octenidine concentration of approx. 0.05 %). The mixture was then homogenized with 1000 ml of water and then extruded through polycarbonate filter pores with the diameter of 100 nm at the temperature of 20° C.
The particle size distribution of the supramolecular aggregate in the obtained suspension was estimated based on the dynamic light scattering technique. The measurements were performed with a laser beam having a wavelength λ=633 nm with a power of 4mW at an the angle of 173 degrees. Samples were analyzed immediately after preparation without dilution. The obtained supramolecular aggregates of phosphatidylcholine and octenidine had an average size of 5.23±0.37 nm with the PDI polydispersity factor of 0.283±0.14.

### Example 5.

4.4 g rapeseed phosphatidylcholine was premixed with 21.0 g of octenidine in the presence of 12.1 g of glycerin (concentration octenidine of approx. 2 %). The mixture was then homogenized with 1000 ml of water and then extruded through polycarbonate filter pores with the diameter of 100 nm at the temperature of 20° C.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. Samples were analyzed immediately after preparation without dilution. The obtained supramolecular aggregates of phosphatidylcholine and octenidine had an average size of 8.16±0.61 nm with the PDI polydispersity factor of 0.321±0.26.

### Example 6.

7.2 g of rapeseed phosphatidylcholine was premixed with 12.1 g of octenidine in the presence of 12.1 g of propane-1,2-diol (octenidine concentration of approx. 1 %). The concentration of phosphatidylcholine is approx. 0.05 %. The mixture was then homogenized with 1000 ml of water and then extruded through polycarbonate filter pores with the diameter of 100 nm at the temperature of 20° C.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. Samples were analyzed immediately after preparation without dilution. The obtained supramolecular aggregates of phosphatidylcholine and octenidine had an average size of 6.84±0.23 nm with the PDI polydispersity factor of 0.198±0.089. Example 7.

53.9 g of soybean phosphatidylcholine 90G was premixed with 12.1 g ofoctenidine in the presence of 12.1 g of propane-1,2-diol (octenidine concentration of approx. 1 %). The concentration of phosphatidylcholine is approx. 5 %.The mixture was then homogenized with 1000 ml of water and then extruded through polycarbonate filter pores with the diameter of 100 nm at the temperature of 20° C.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. Samples were analyzed immediately after preparation without dilution. The obtained supramolecular aggregates of phosphatidylcholine and octenidine had an average size of 9.04±0.34 nm with the PDI polydispersity factor of 0.296±0.13.

### Example 8.

14.4 g of soybean phosphatidylcholine 90G was premixed with 12.1 g of octenidine in the presence of 10.3 g of glycerin (concentration octenidine of approx.1 %). The mixture was then homogenized with 1000 ml of water and then extruded through polycarbonate filter pores with the diameter of 100 nm at the temperature of 20° C.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. Samples were analyzed immediately after preparation without dilution. The obtained supramolecular aggregates of phosphatidylcholine and octenidine had an averages size of 5.84±0.14 nm with the PDI polydispersity factor of 0.168±0.012.

### Example 9.

14.4 g of soybean phosphatidylcholine 90G was premixed with 12.1 g of octenidine (octenidine concentration of approx. 1 %) in the presence of 256.5 g propane-1,2-diol (concentration of propane-1,2-diol of approx. 20 %). The concentration of phosphatidylcholine is approx. 5%. The mixture was then homogenized with 1000 ml of water and then extruded through polycarbonate filter pores with the diameter of 100 nm at the temperature of 20° C.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. Samples were analyzed immediately after preparation without dilution. The obtained supramolecular aggregates of phosphatidylcholine and octenidine had an average size of 5.12± 0.08 nm with the PDI polydispersity factor of 0.143±0.009.

### Example 10.

14.4 g of soybean phosphatidylcholine 90G was premixed with 12.1 g of octenidine in the presence of 12.1 g of propane-1,2-diol (octenidine concentration of approx. 1 %). The mixture was then homogenized with 1000 ml of water and then extruded through polycarbonate filter pores with the diameter of 100 nm at the temperature of 40° C.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. Samples were analyzed immediately after preparation without dilution. The obtained supramolecular aggregates of phosphatidylcholine and octenidine had an average size of 4.38±0.11 nm with the PDI polydispersity factor of 0.198±0.007.

### Example 11.

14.4 g of soybean phosphatidylcholine 90G was premixed with 12.1 g of octenidine in the presence of 12.1 g of propane-1,2-diol (octenidine concentration of approx. 1 %). The mixture was then homogenized with 1000 ml of water and then extruded through polycarbonate filter pores with the diameter of 100 nm at the temperature of 40° C. The suspension of extruded supramolecular aggregates was then mixed with agelling agent, Carbopol^{®}934 (acrylic acid polymer) in amount of 0.5 % and methyl parahydroxybenzoate preservative in the amount of 0.1 %.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. In the measuring process a supernatant was used which was obtained by centrifugation of the gelled suspension of supramolecular aggregates using Carbopol^{®}934. The obtained supramolecular aggregates of phosphatidylcholine and octenidine had an average size of 3.94± 0.04 nm with the PDI polydispersity factor of 0.208± 0.06. This also shows that gelling of the suspension of supramolecular aggregates with Carbopol^{®}934 does not change their topology.

### Example 12.

24.1 g of rapeseed phosphatidylcholine was premixed with 11.9 g of octenidine (octenidine concentration is 0.2%) in the presence of 24.1g of propane-1,2-diol. The mixture was then homogenized with 1000 ml of water and then extruded through polycarbonate filter pores with the diameter of 100 nm at the temperature of 20° C.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. The obtained supramolecular aggregates of phosphatidylcholine and octenidine had an average size of 3.60± 0.04 nm with the PDI polydispersity factor of 0.241±0.005.

### Example 13.

14.4 g of soybean phosphatidylcholine 90G was premixed with 12.1 g of octenidine in the presence of 12.1 g of propane-1,2-diol. The mixture was then treated with pH 9.0, and then extruded through polycarbonate filter pores with the diameter of 100 nm at the temperature of 25° C.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. The obtained supramolecular aggregates of phosphatidylcholine and octenidine had an average size of 3.678± 0.02 nm with the PDI polydispersity factor of 0.151±0.004.

### Example 14.

14.4 g of soybean phosphatidylcholine 90G was premixed with 12.1 g of octenidine in the presence of 12.1 g of propane-1,2-diol. The mixture was then treated with a solution containing 10 mM of Hepes buffer in 1000 ml of water by adjusting the pH to 9.0, then extruded through polycarbonate filter pores with the diameter of 100 nm at the temperature of 25° C.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. The obtained supramolecular aggregates of phosphatidylcholine and octenidine had an average size of 4.96±0.1601 nm with the PDI polydispersity factor of 0.165±0.04.

### Example 15.

14.41 g of soybean phosphatidylcholine 90G was premixed with 12.1 g octenidine in the presence of 12.1 g of propane-1,2-diol. The mixture was then treated with a solution containing 10 mM of a buffer SSA (1-hydroxy-4-sulfobenzoic) in 1000 ml of water by adjusting the pH to 9.0, then extruded through polycarbonate filter pores with the diameter of 100 nm at the temperature of 25° C. The supramolecular aggregate suspension obtained was then mixed with NaCl, so that the final NaCl concentration equaled 140 mM.

The particle size distribution of the supramolecular aggregate in the obtained suspension was estimated based on the dynamic light scattering technique. The measurements were performed with a laser beam having a wavelength λ=633 nm with a power of 4mW at the angle of 173 degrees. The obtained supramolecular aggregates of phosphatidylcholine and octenidine had an average size of 4.96±0.1601 nm with the PDI polydispersity factor of 0.165±0.04.

The obtained results show that the size of supramolecular aggregates composed of phosphatidylcholine and octenidine range from 3-20 nm, which proves that they are not liposomes. The bactericide preparation made on the basis of a supramolecular aggregate, according to the invention, shows no irritant properties and does not cause allergic reactions, as occursin preparations containing significant amounts of alcohol and/or detergents.

Octenidine located in a supramolecular aggregate at the concentration of 0.1 % meets all the criteria required for disinfectant preparations described in the Polish norm PN-EN123 727+ A1 entitled: "Chemical disinfectants and antiseptics -Quantitative suspension test for the evaluation of bactericidal activity in the medical area- Test method and requirements ". The supramolecular aggregate, according to the invention, as an ingredient of bactericides, is suitable for application to irritated skin, wounds and mucous membranes of humans and animals. It is designed for general use in places where septic conditions should be maintained, such as health centers, food establishments and other foodservice facilities, pharmaceuticals plants, cosmetics plants and food processing plants.

## Claims

1. Supramolecular aggregate in the form of phosphatidylcholine and octenidine complex, wherein the molar ratio of octenidine to phosphatidylcholine ranges from 1:2 to 2:1, octenidine concentration equals from 0.05 to 2 % by weight and the concentration of phosphatidylcholine equals 0.05 to 5 % by weight.

2. Aggregate, according to claim 1, **characterized in that** phosphatidylcholine comprises phosphatidylcholine selected from the group consisting of soybean phosphatidylcholine, rapeseed phosphatidylcholine, hen's eggs phosphatidylcholine.

3. A pharmaceutical composition, **characterized in that** it is in the form of suspension comprising a supramolecular aggregate of phosphatidylcholine and octenidine dissolved in polyhydric alcohol of low molecular weight, wherein the molar ratio of octenidine to phosphatidyl choline ranges from 1:2 to 2:1, the concentration of octenidine equals from 0.05 to 2 % by weight and the concentration of phosphatidylcholine equals 0.05 to 5 % by weight.

4. A composition according to claim 3, **characterized in that** the concentration of polyhydric alcohol of low molecular weight equals from 1 to 20 % by weight.

5. A composition according to claim 3 or 4, **characterized in that** propane-1,2-diol or glycerol is used as polyhydric alcohol of low molecular weight.

6. A pharmaceutical composition, **characterized in that** it is in the form of a gel comprising a supramolecular aggregate of phosphatidylcholine and octenidine dissolved in polyhydric alcohol of low molecular weight, wherein the molar ratio of octenidine to phosphatidylcholine ranges from 1: 2 to 2: 1, the concentration of octenidine equals from 0.05 to 2 % by weight and the concentration of phosphatidylcholine equals 0.05 to 5 % by weight, and the gelling agent is an acrylic acid polymer.

7. The composition according to claim 6, **characterized in that** it comprises a preservative, preferably methyl parahydroxybenzoate.

8. The composition according to claim 6, **characterized in that** the concentration of polyhydric alcohol of low molecular weight equals from 1 to 20 % by weight.

9. The composition according to claim 6 or 8, **characterized in that** propane-1,2-diol or glycerol is used as polyhydric alcohol of low molecular weight.

10. The method for preparing a supramolecular aggregate in the form of a complex of phosphatidylcholine and octenidine, wherein the molar ratio of octenidine to phosphatidylcholine ranges from 1: 2 to 2: 1, the concentration of octenidine equals from 0.05 to 2 % by weight and the concentration of phosphatidylcholine equals 0.05 to 5 % by weight, **characterized in that** octenidine is mixed with phosphatidylcholine in the presence of polyhydric alcohol of low molecular weight, after which the entire composition is homogenized with water and/or water with a buffer, and then extruded through a polycarbonate filter pores with the diameter of 50-400 nm at the temperature range of 20° to 40° C.

11. The method according to claim 10, **characterized in that** a polycarbonate filter with a pore diameter of 100 mm is used.

12. The method according to claim 10, **characterized in that** the buffer used is selected from the group comprising triethanolamine and biological buffers.

13. The method according to claim 10, **characterized in that** the concentration of polyhydric alcohol of low molecular weight equals from 1 to 20 % by weight.

14. The method according to claim10 or 13, **characterized in that** propane-1,2-diol or glycerol is used as polyhydric alcohol of low molecular weight.

15. The method according to claim 10 or 12, **characterized in that** the biological buffer used is selected from from the group consisting of 2- [4- (2-hydroxyethyl) -1-piperazinyl] ethanesulfonic acid, 2-morpholineoethanesulfonic acid, 3-[N-morpholino]propane-sulfonic acid, piperazine-1,4-bis[2-ethanesulfonic] acid, N-[Tris(hydroxymethyl)methyl]-2-aminoethanesulfonic acid, 1-hydroxy-4-sulfobenzoic *acid.*

16. The method according to claim 10, **characterized in that** the concentration of the buffer does not exceed 5 % by weight.
